# EUROPEAN PATENT SPECIFICATION

(11) **EP 3 252 466 B1**
(45) Date of publication and mention of the grant of the patent: **07.08.2019**
(21) Application number: 15879958.5
(22) Date of filing: 29.01.2015
(51) Int. Cl.: G01N 33/36, G09B 23/28

(54) **SWEAT SIMULATOR AND SWEAT SIMULATION METHOD**
SCHWEISSSIMULATOR UND SCHWEISSSIMULATIONSVERFAHREN
SIMULATEUR D'EXSUDATION ET PROCÉDÉ DE SIMULATION D'EXSUDATION

(43) Date of publication of application: 06.12.2017
(73) Proprietor: Kaken Test Center, Tokyo 103-0022 (JP)
(72) Inventor: YASUDA, Atsushi, Tokyo 1030022 (JP)
(74) Representative: Rüger Abel
(86) International application number: PCT/JP2015/052558
(87) International publication number: WO 2016/121061

(56) References cited:
- WO-A1-2011/122033
- JP-A- H0 970 422
- JP-A- 2003 167 510
- JP-A- 2008 046 048
- JP-U- 3 121 969
- KIMIHIRO ADACHI: 'Wet Skin Simulator' JOURNAL OF THE SOCIETY OF FIBER SCIENCE AND TECHNOLOGY vol. 52, no. 10, 01 January 1996, pages 403 - 408, XP055463900

## Description

### Technical Field

The present disclosure relates to a device and method for simulating perspiration of a human body.

### Background Art

Heat and moisture produced in a human body are constantly released into the environment system thorough the clothing. Characteristics of transfer of heat and moisture from a human body to the environmental system through the clothing are important factors affecting whether a person feels comfortable. Particularly, the temperature/humidity within clothing is called "the environment within clothing."

Perspiration of a human body is classified into two types: the liquid phase perspiration producing liquid sweat as the temperature of the body and/or the environment rises and the vapor phase perspiration that is evaporation of moisture from the skin surface due to the difference in water vapor pressure between the body surface and the environment. The vapor phase perspiration is also called insensible perspiration and the person is unaware of the evaporation from the skin. The quantity of vapor phase perspiration varies depending on the body part, the skin temperature, the environmental temperature/humidity, and the like and is said to be approximately 20 g/hr/m². Along with the liquid phase perspiration, the vapor phase perspiration also affects the environment within clothing.

As a device checking the warmth retaining property of an item of clothing, there is a warmth retaining property measuring device using a constant-temperature exothermic body assumed to be a skin and measuring the quantity of heat necessary for keeping the exothermic body covered with a sample at 36 °C in an environment of 20 °C and 65 % RH. This is a measuring device called "a skin model" and such a device additionally provided with a mechanism simulating the perspiration is generally used.

JP 2008046048 A and JP 3121969 U disclose devices measuring characteristics of transfer of heat and water vapor in a steady state of textile products or the like.

WO 2011/122033 A1 discloses an artificial perspiration apparatus comprising a porous plate provided horizontally and comprising vapor permeable holes allowing vapor to pass through, and a uniform heat block fixed in contact with an underside of the porous plate and comprising vapor passages in communication with the vapor permeable holes for passing vapor thereto. The vapor passages may be tubes inserted into and running through the heat block or through holes formed therethrough, the tubes or through holes having a uniform diameter in vertical direction throughout the heat block. A heating device heats the uniform heat block, and a vapor supply device supplies vapor through the vapor passages to the vapor permeable holes. The vapor supply device comprises a vapor generating portion that generates water-saturated vapor, a pump that supplies water to the vapor generating portion, a dry air generating portion that generates and supplies dry air to the vapor generating portion, and a flow rate / pressure measurement instrument that measures a flow rate or pushing pressure of the vapor that is pushed from the vapor generation portion to the vapor passages of the uniform heat block. A control device controls the driving of the artificial perspiration apparatus and the amount of the vapor discharged from the vapor permeable holes of the porous plate by adjusting the amount of the water supplied to the vapor generating portion by controlling driving the pump of the vapor supply device and controlling the amount of the dry air supplied to the vapor generating portion.

### Summary of Invention

### Technical Problem

The devices described in JP 2008046048 A and JP 3121969 U cover an electrically heated porous metal plate with a moisture-permeable waterproof film allowing water vapor to pass through but not allowing liquid water to pass through for measuring the vapor resistance. Moisture supplied to the heated porous metal plate evaporates and passes through the film as vapor. However, the above devices allow for a large quantity of evaporation compared to the vapor phase perspiration of a human body and are incapable of adjusting the quantity of evaporation. The artificial perspiration apparatus described in WO 2011/122033 A1 is capable of adjusting the quantity of evaporation, but using a rather complex structure.

The present disclosure is made with the view of the above situation and an objective of the disclosure is to make it possible to simulate the vapor phase perspiration of a human body and adjust the quantity of evaporation.

### Solution to Problem

In order to achieve the above objective, the perspiration simulator according to the present disclosure comprises:
a porous plate provided horizontally and allowing vapor to pass through;
a uniform heat block fixed in contact with an underside of the porous plate and comprising one or more open top cavities of which the area in cross-section parallel to the underside of the porous plate changes in a vertical direction;
a heating device heating the uniform heat block; and
a first water supply device supplying water to the cavities and controlling a water level in the cavities.

The perspiration simulation method according to the present disclosure
supplies water to one or more open top cavities of a uniform heat block that is fixed in contact with an underside of a porous plate provided horizontally and allowing vapor to pass through and is heated by a heating device, and changes an area of a water surface in the cavities to adjust a quantity of evaporation from the porous plate.

### Advantageous Effects of Invention

The present disclosure makes it possible to simulate the vapor phase perspiration of a human body and adjust the quantity of evaporation.

### Brief Description of Drawings

FIG. 1 is a block diagram showing an exemplary configuration of the perspiration simulator according to Embodiment 1 of the present disclosure;
FIG. 2 is an illustration showing an example of the cavities of the uniform heat block of the perspiration simulator according to Embodiment 1;
FIG. 3 is a chart showing change in the quantity of evaporation from the porous plate in association with change in the water level in the cavities;
FIG. 4 is a block diagram showing an exemplary configuration of the perspiration simulator according to Embodiment 2 of the present disclosure;
FIG. 5 is an illustration showing an example of the watering path of the perspiration simulator according to Embodiment 2; and
FIG. 6 is a block diagram showing an exemplary configuration of the perspiration simulator according to Embodiment 3 of the present disclosure.

### Description of Embodiments

Embodiments of the present disclosure will be described in detail below with reference to the drawings. In the figures, the same or corresponding parts are referred to by the same reference signs.

### Embodiment 1

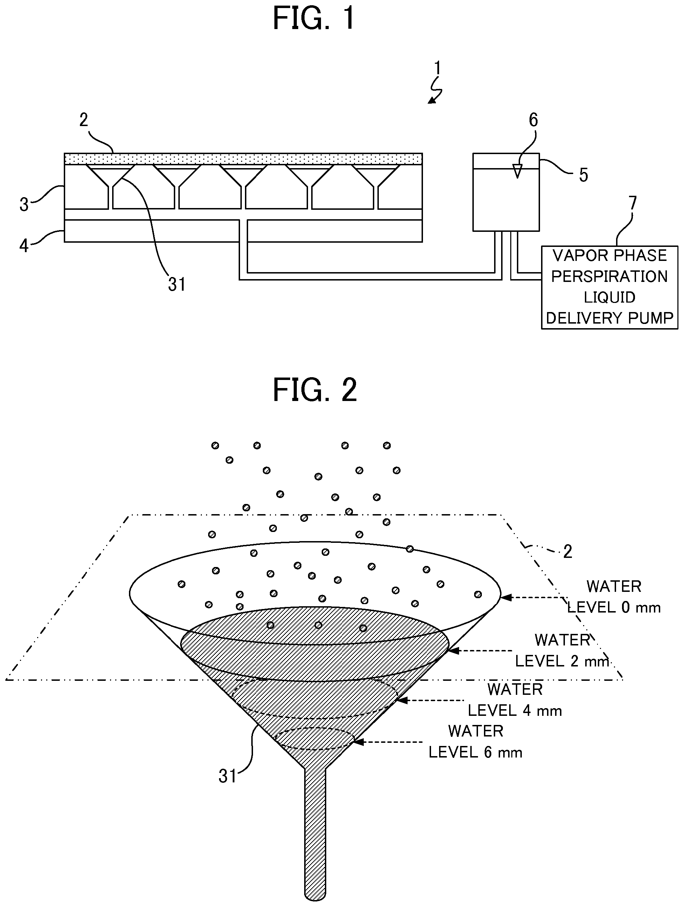

FIG. 1 is a block diagram showing an exemplary configuration of the perspiration simulator according to Embodiment 1 of the present disclosure. A perspiration simulator 1 comprises a porous plate 2, a uniform heat block 3, a heating device 4, a vapor phase perspiration tank 5, a water level sensor 6, and a vapor phase perspiration liquid delivery pump 7. The porous plate 2 is a plate formed by a porous body of sintered metal powder or a porous ceramic in which fine air holes are formed. The porous plate 2 is horizontally placed and allows vapor to pass through.

The uniform heat block 3 is formed by a highly heat-conductive material, for example metal such as aluminum or copper. The uniform heat block 3 is fixed in contact with the underside of the porous plate 2 and has one or more cavities 31. The cavities 31 each have the area in cross-section parallel to the underside of the porous plate 2 changed in the vertical direction and open up. The heating device 4 heats the uniform heat block 3 to a given temperature (for example, 36 °C). The vapor phase perspiration tank 5 holds water to supply to the cavities 31. The water level sensor 6 detects the water level of water in the vapor phase perspiration tank 5. The vapor phase perspiration liquid delivery pump 7 supplies water in the vapor phase perspiration tank 5 to the cavities 31 and controls the water level in the cavities 31.

In the example of FIG. 1, the uniform heat block 3 has a total of 25, five lengthwise by five widthwise, cavities 31. The porous plate 2 is fixed in contact with the top surface of the uniform heat block 3 so as to cover all cavities 31. As water in the vapor phase perspiration tank 5 is supplied by the vapor phase perspiration liquid delivery pump 7 into the cavities 31 of the uniform heat block 3 heated by the heating device 4, the water evaporates from the water surface in the cavities 31 and the vapor passes through the porous plate 2.

FIG. 2 is an illustration showing an example of the cavities of the uniform heat block of the perspiration simulator according to Embodiment 1. The cavities 31 each have a cone shape of which the axis of revolution is oriented in the vertical direction, open up, and have a water supply hole at the bottom. The water supply holes of the cavities 31 and the vapor phase perspiration tank 5 are connected by communication tubes and the water levels in the vapor phase perspiration tank 5 and the cavities 31 change in connection with each other. The vapor phase perspiration liquid delivery pump 7 drives a pump so that the water level detected by the water level sensor 6 reaches a prescribed value, whereby controlling the water level in the cavities 31. The vapor phase perspiration liquid delivery pump 7 supplies water in an amount covering the evaporation to maintain a constant water level.

The water level in the cavities 31 is measured downward in the vertical direction as being positive from the surface in contact with the porous plate 2 at 0 mm. Having a cone shape of which the axis of revolution is oriented in the vertical direction, the cavities 31 have a larger water surface area when the value of the water level is lower. As a result, it is possible to control the quantity of evaporation from the water surface in the cavities 31 and adjust the quantity of evaporation from the porous plate 2 allowing the vapor from the cavities 31 to pass through. Here, the water supplied to the cavities 31 is set to a given temperature (for example, 36 °C).

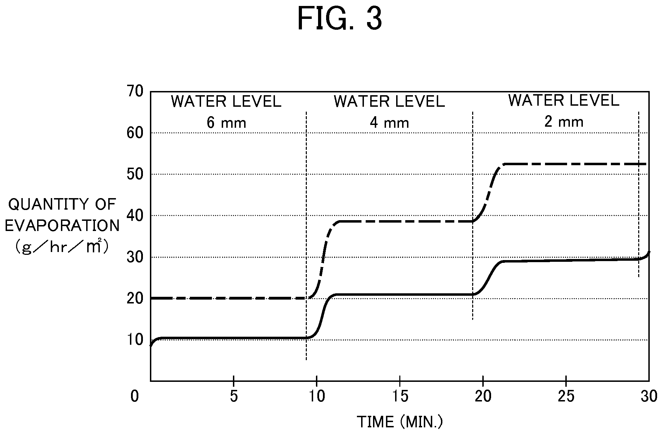

FIG. 3 is a chart showing change in the quantity of evaporation from the porous plate in association with change in the water level in the cavities. FIG. 3 is a graphical representation with the quantity of evaporation from the porous plate 2 plotted as ordinate and the time plotted as abscissa. The full line is a graph when the porous plate 2 having a nominal filtration diameter of 40 µm is used. The alternate long and short dash line is a graph when the porous plate 2 having a nominal filtration diameter of 100 µm is used. Moreover, the quantity of evaporation from the porous plate 2 is measurements in an environment at the temperature of 20 °C and the relative humidity of 65 % and with the wind speed of 0.5 m/min.

As the water level in the cavities 31 is changed to 6 mm, 4 mm, and 2 mm (see FIG. 2), the quantity of evaporation from the porous plate 2 changes as presented by the graphs shown in FIG. 3. In the example of FIG. 3, the perspiration simulator 1 can realize the quantity of evaporation nearly equal to insensible perspiration (around 20 g/hr/m²) by setting the water level in the cavities 31 to approximately 4 mm when the porous plate 2 having a nominal filtration diameter of 40 µm is used and setting the water level in the cavities 31 to approximately 6 mm when the porous plate 2 having a nominal filtration diameter of 100 µm is used, and can simulate the vapor phase perspiration of a human body.

The perspiration simulator 1 according to Embodiment 1 makes it possible to simulate the vapor phase perspiration of a human body and adjust the quantity of evaporation. Moreover, since the cavities 31 have a cone shape of which the axis of revolution is oriented in the vertical direction, the cavities 31 of the uniform heat block 3 are easily processed. Here, the lateral surface of the cavities 31 may be a paraboloid of revolution of which the axis of revolution is oriented in the vertical direction. In such a case, the area of the water surface is expressed by a linear function of the water level, whereby it is easy to adjust the area of the water surface. In any case, the area in cross-section (the water surface) parallel to the underside of the porous plate monotonically increases in the vertically upward direction.

### Embodiment 2

The perspiration simulator 1 according to Embodiment 2 simulates both the vapor phase perspiration and the liquid phase perspiration of a human body.

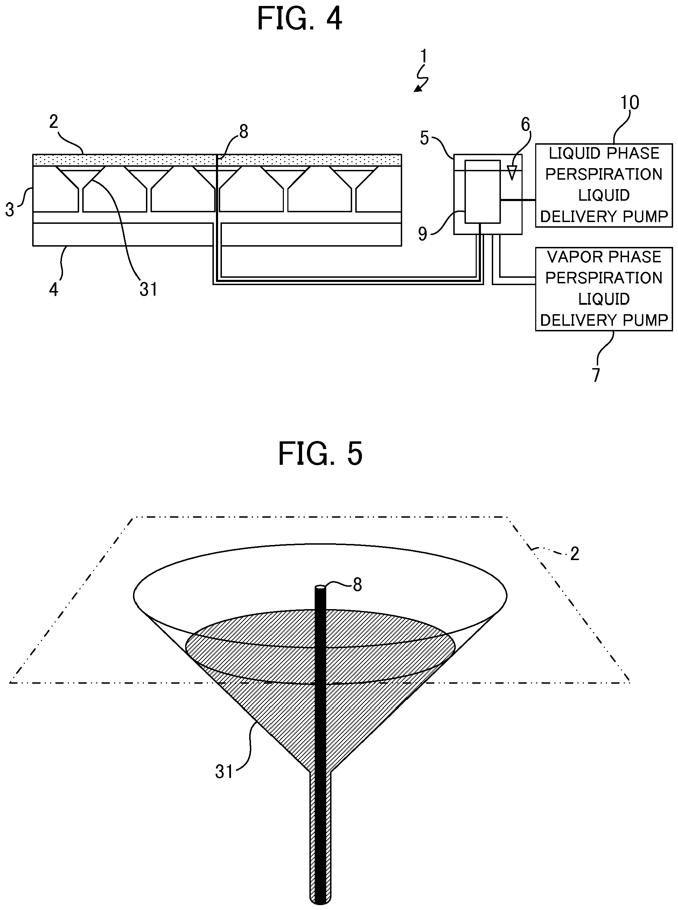

FIG. 4 is a block diagram showing an exemplary configuration of the perspiration simulator according to Embodiment 2 of the present disclosure. The perspiration simulator 1 according to Embodiment 2 comprises, in addition to the configuration of the perspiration simulator 1 of Embodiment 1, a watering path 8, a liquid phase perspiration tank 9, and a liquid phase perspiration liquid delivery pump 10. The watering path 8 runs within the uniform heat block 3 and penetrates the porous plate 2 in the vertical direction. The liquid phase perspiration tank 9 holds water to supply to the watering path 8. The liquid phase perspiration liquid delivery pump 10 supplies water in the liquid phase perspiration tank 9 to the watering path 8 so as to supply water to the top surface of the porous plate 2. The liquid phase perspiration liquid delivery pump 10 can adjust the quantity of water supplied to the top surface of the porous plate 2 by adjusting the quantity of water to supply to the watering path 8.

The watering path 8 may run through a cavity 31 of the uniform heat block 3 as shown in FIG. 4, or may run through somewhere other than the cavities 31 of the uniform heat block 3. Moreover, the watering path 8 is not restricted to only one path. When multiple watering paths 8 are provided, it is favorable to provide the openings of the watering paths 8 in a point symmetric pattern around the center of the porous plate 2. In the example of FIG. 4, the liquid phase perspiration tank 9 is disposed in the vapor phase perspiration tank 5. However, this is not restrictive and the liquid phase perspiration tank 9 may be disposed outside the vapor phase perspiration tank 5.

FIG. 5 is an illustration showing an example of the watering path of the perspiration simulator according to Embodiment 2. In the examples of FIGS. 4 and 5, the watering path 8 extends within the cavity 31 of the uniform heat block 3 in the vertical direction and penetrates the porous plate 2. With such a configuration, there is no need of forming a new cavity in the uniform heat block 3 for the watering path 8. Here, water supplied to the watering path 8 is set to nearly the same as the human body temperature like the water supplied to the cavities 31.

The perspiration simulator 1 according to Embodiment 2 makes it possible to simulate both the vapor phase perspiration and the liquid phase perspiration of a human body with one device by supplying water to the watering path 8 running within the uniform heat block 3 and penetrating the porous plate 2 in the vertical direction and adjusting the quantity of water supplied to the top surface of the porous plate 2 in addition to the configuration of Embodiment 1. Here, as in Embodiment 1, the cavities 31 may have a cone shape of which the axis of revolution is oriented in the vertical direction or may have a shape of which the lateral surface is a paraboloid of revolution of which the axis of revolution is oriented in the vertical direction.

### Embodiment 3

In Embodiment 3, using a textile product such as an item of clothing as a sample, the quantity of released heat while the porous plate 2 of the perspiration simulator 1 is covered with the sample is measured.

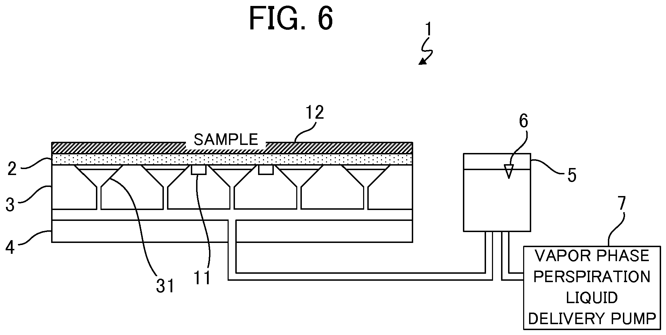

FIG. 6 is a block diagram showing an exemplary configuration of the perspiration simulator according to Embodiment 3 of the present disclosure. The perspiration simulator 1 of Embodiment 3 comprises a heat flux sensor 11 between the porous plate 2 and the uniform heat block 3 in addition to the configuration of the perspiration simulator 1 of Embodiment 1. The heat flux sensor 11 measures the quantity of heat crossing a unit area in the vertical direction in a unit time. With the porous plate 2 of the perspiration simulator 1 being covered with a sample 12, it is possible to examine the warmth retaining property of the sample 12 during the vapor phase perspiration of a human body.

In addition to the configuration of the perspiration simulator 1 of Embodiment 2, the heat flux sensor 11 may be provided between the porous plate 2 and the uniform heat block 3. In such a case, with the porous plate 2 of the perspiration simulator 1 being covered with the sample 12, it is possible to examine the warmth retaining property of the sample 12 during the vapor phase perspiration and/or the liquid phase perspiration of a human body. Moreover, it is possible to examine the aeration property and the moisture absorption property of the sample 12 during the vapor phase perspiration and/or the liquid phase perspiration of a human body by measuring the quantity of evaporation while the porous plate 2 of the perspiration simulator 1 is covered with the sample 12.

The perspiration simulator 1 according to Embodiment 3 makes it possible to examine the warmth retaining property of the sample 12 during the vapor phase perspiration and/or the liquid phase perspiration of a human body with one device by using a textile product such as an item of clothing as the sample 12 and measuring the quantity of released heat while the porous plate 2 is covered. The measurement of the quantity of released heat is not restricted to by the heat flux sensor 11. The quantity of power to maintain the uniform heat block 3 at a constant temperature (for example, 36 °C) may be measured as the quantity of released heat. When the quantity of released heat is measured using the heat flux sensor 11, it is possible to measure the quantity of released heat with high accuracy with no auxiliary device such as a guard heater preventing heat release in the horizontal direction in order to release heat from the heating device 4 in the vertical direction. Here, the perspiration simulator 1 may comprise a measuring device measuring the quantity of power to maintain the uniform heat block 3 at a constant temperature as the quantity of released heat. Measuring the quantity of released heat using the heat flux sensor 11 is a quicker way than measuring the quantity of power to maintain the uniform heat block 3 at a constant temperature as the quantity of released heat.

The cavities 31 are not restricted in shape to, as in the above-described embodiments, a cone shape of which the axis of revolution is oriented in the vertical direction or a shape of which the lateral surface is a paraboloid of revolution of which the axis of revolution is oriented in the vertical direction. It is sufficient that the cross-section of the cavities 31 parallel to the top surface (the underside of the porous plate 2) is given by a function of the distance from the top surface and the area of the water surface is controllable by adjusting the water level. However, it is preferable that the cavities 31 have a shape of which the area in cross-section parallel to the underside of the porous plate 2 monotonically increases and opening up. The monotonic increase in this case is the monotonic increase in the broad sense of the term and may include a part constant in the cross-sectional area. Furthermore, for example, the uniform heat block 3 may be elastic and the cavities 31 may change their shape.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents to which such claims are entitled.

### Reference Signs List

- 1: Perspiration simulator
- 2: Porous plate
- 3: Uniform heat block
- 4: Heating device
- 5: Vapor phase perspiration tank
- 6: Water level sensor
- 7: Vapor phase perspiration liquid delivery pump
- 8: Watering path
- 9: Liquid phase perspiration tank
- 10: Liquid phase perspiration liquid delivery pump
- 11: Heat flux sensor
- 12: Sample
- 31: Cavity

## Claims

1. A perspiration simulator (1), **characterized by** comprising:
a porous plate (2) provided horizontally and allowing vapor to pass through;
a uniform heat block (3) fixed in contact with an underside of the porous plate (2) and comprising one or more open top cavities (31) of which the area in cross-section parallel to the underside of the porous plate (2) changes in a vertical direction;
a heating device (4) heating the uniform heat block (3); and
a first water supply device (5, 6, 7) supplying water to the cavities (31) and controlling a water level in the cavities (31).

2. The perspiration simulator (1) according to claim 1, **characterized in that**
the uniform heat block (3) comprises water supply holes at bottoms of the cavities (31).

3. The perspiration simulator (1) according to claim 1 or 2, **characterized in that**
the change in the vertical direction of the area in cross-section parallel to the underside of the porous plate (2) of the cavities (31) of the uniform heat block (3) is of monotonic increase in an upward direction.

4. The perspiration simulator (1) according to claim 3, **characterized in that**
the cavities (31) of the uniform heat block (3) are in a cone shape of which an axis of revolution is oriented in the vertical direction.

5. The perspiration simulator (1) according to claim 3, **characterized in that**
a lateral surface of the cavities (31) of the uniform heat block (3) is a paraboloid of revolution of which an axis of revolution is oriented in the vertical direction.

6. The perspiration simulator (1) according to any one of claims 1 to 5, **characterized by** further comprising a second water supply device (8, 9, 10) supplying water to a top surface of the porous plate (2).

7. The perspiration simulator (1) according to claim 6, **characterized in that**
the second water supply device (8, 9, 10) includes a watering path (8) running within the uniform heat block (3) and penetrating the porous plate (2) in the vertical direction and controls water supply to the top surface of the porous plate (2) by adjusting a quantity of supplied water to the watering path (8).

8. The perspiration simulator (1) according to claim 7, **characterized in that**
the watering path (8) of the second water supply device (8, 9, 10) runs within the cavities (31) of the uniform heat block (3).

9. The perspiration simulator (1) according to any one of claims 1 to 8, **characterized by**
comprising a heat flux sensor (11) provided between the porous plate (2) and the uniform heat block (3) and measuring a quantity of heat crossing a unit area in the vertical direction in a unit time.

10. The perspiration simulator (1) according to any one of claims 1 to 9, **characterized in that**
the heating device (4) comprises a measuring device measuring a quantity of power maintaining the uniform heat block (3) at a constant temperature as a quantity of released heat.

11. A perspiration simulation method supplying water to one or more open top cavities (31) of a uniform heat block (3) that is fixed in contact with an underside of a porous plate (2) provided horizontally and allowing vapor to pass through and is heated by a heating device (4), and changing an area of a water surface in the cavities (31) to adjust a quantity of evaporation from the porous plate (2).

## Patentansprüche

1. Schwitzsimulator (1), **dadurch gekennzeichnet, dass** er aufweist:
eine poröse Platte (2), die horizontal vorgesehen ist und Dampf passieren lässt;
einen gleichmäßigen Wärmeblock (3), der mit einer Unterseite der porösen Platte (2) in Kontakt stehend fixiert ist und eine oder mehrere offene obere Hohlräume (31) aufweist, von denen die Querschnittsfläche parallel zu der Unterseite der porösen Platte (2) sich in einer vertikalen Richtung verändert;
eine Heizvorrichtung (4), die den gleichmäßigen Wärmeblock (3) erwärmt; und
eine erste Wasserzufuhrvorrichtung (5, 6, 7), die Wasser den Hohlräumen (31) zuführt und einen Wasserstand in den Hohlräumen (31) steuert.

2. Schwitzsimulator (1) nach Anspruch 1, **dadurch gekennzeichnet, dass**
der gleichmäßige Wärmeblock (3) Wasserzufuhrlöcher an den Böden der Hohlräume (31) aufweist.

3. Schwitzsimulator (1) nach Anspruch 1 oder 2, **dadurch gekennzeichnet, dass**
die Veränderung der Querschnittsfläche parallel zu der Unterseite der porösen Platte (2) der Hohlräume (1) des gleichmäßigen Wärmeblocks (3) in der vertikalen Richtung von einer monotonen Zunahme in einer Aufwärtsrichtung ist.

4. Schwitzsimulator (1) nach Anspruch 3, **dadurch gekennzeichnet, dass**
die Hohlräume (31) des gleichmäßigen Wärmeblocks (3) eine konische Form aufweisen, deren Rotationsachse in der vertikalen Richtung ausgerichtet ist.

5. Schwitzsimulator (1) nach Anspruch 3, **dadurch gekennzeichnet, dass**
eine Seitenfläche der Hohlräume (31) des gleichmäßigen Wärmeblocks (3) ein Rotationsparaboloid ist, dessen Rotationsachse in der vertikalen Richtung ausgerichtet ist.

6. Schwitzsimulator nach einem beliebigen der Ansprüche 1 bis 5, **dadurch gekennzeichnet, dass** er ferner eine zweite Wasserzufuhrvorrichtung (8, 9, 10) aufweist, die Wasser einer oberen Oberfläche der porösen Platte (2) zuführt.

7. Schwitzsimulator (1) nach Anspruch 6, **dadurch gekennzeichnet, dass**
die zweite Wasserzufuhrvorrichtung (8, 9, 10) einen Bewässerungsweg (8) enthält, der innerhalb des gleichmäßigen Wärmeblocks (3) verläuft und die poröse Platte (2) in der vertikalen Richtung durchdringt und die Wasserzufuhr zu der oberen Oberfläche der porösen Platte (2) durch Einstellung einer Menge des dem Bewässerungsweg (8) zugeführten Wassers steuert.

8. Schwitzsimulator (1) nach Anspruch 7, **dadurch gekennzeichnet, dass**
der Bewässerungsweg (8) der zweiten Wasserzufuhrvorrichtung (8, 9, 10) innerhalb der Hohlräume (31) des gleichmäßigen Wärmeblocks (3) verläuft.

9. Schwitzsimulator (1) nach einem beliebigen der Ansprüche 1 bis 8, **dadurch gekennzeichnet, dass**
er einen Wärmeflusssensor (11) aufweist, der zwischen der porösen Platte (2) und dem gleichmäßigen Wärmeblock (3) vorgesehen ist und eine Wärmemenge misst, die eine Einheitsfläche in der vertikalen Richtung in einer Zeiteinheit durchquert.

10. Schwitzsimulator (1) nach einem beliebigen der Ansprüche 1 bis 9, **dadurch gekennzeichnet, dass**
die Heizvorrichtung (4) eine Messvorrichtung aufweist, die eine Leistungsmenge, die den gleichmäßigen Wärmeblock (3) auf einer konstanten Temperatur hält, als eine Menge der abgegebenen Wärme misst.

11. Schwitzsimulationsverfahren, das Wasser einer oder mehreren offenen oberen Hohlräumen (31) eines gleichmäßigen Wärmeblocks (3) zuführt, der mit einer Unterseite einer porösen Platte (2) in Kontakt stehend fixiert ist, die horizontal vorgesehen ist und Dampf passieren lässt und die durch eine Heizvorrichtung (4) erwärmt wird, und das eine Fläche einer Wasseroberfläche in den Hohlräumen (31) verändert, um eine Menge der Verdunstungsmenge aus der porösen Platte (2) einzustellen.

## Revendications

1. Simulateur de transpiration (1), **caractérisé en ce qu'**il comprend :
une plaque poreuse (2), prévue horizontalement et permettant à la vapeur de passer au travers ;
un bloc chauffant uniforme (3), fixé en contact avec un côté inférieur de la plaque poreuse (2) et comprenant une ou plusieurs cavités supérieures ouvertes (31), dont la superficie en coupe transversale, parallèle au côté inférieur de la plaque poreuse (2), change dans une direction verticale ;
un dispositif de chauffage (4), qui chauffe le bloc chauffant uniforme (3) et
un premier dispositif d'alimentation en eau (5, 6, 7), qui amène de l'eau aux cavités (31) et qui commande un niveau d'eau dans les cavités (31).

2. Simulateur de transpiration (1) selon la revendication 1,
**caractérisé en ce que**
le bloc chauffant uniforme (3) comprend des trous d'alimentation en eau, au fond des cavités (31).

3. Simulateur de transpiration (1) selon la revendication 1 ou 2,
**caractérisé en ce que**
le changement dans la direction verticale de la superficie en coupe transversale, parallèle au côté inférieur de la plaque poreuse (2) des cavités (31) du bloc chauffant uniforme (3), suit une augmentation monotone dans une direction verticale.

4. Simulateur de transpiration (1) selon la revendication 3,
**caractérisé en ce que**
les cavités (31) du bloc chauffant uniforme (3) sont de forme conique, dont un axe de révolution est orienté dans la direction verticale.

5. Simulateur de transpiration (1) selon la revendication 3,
**caractérisé en ce**
**qu'**une surface latérale des cavités (31) du bloc chauffant uniforme (3) est un paraboloïde de révolution, dont un axe de révolution est orienté dans la direction verticale.

6. Simulateur de transpiration (1) selon l'une quelconque des revendications 1 à 5,
**caractérisé en ce**
**qu'**il comprend en outre un second dispositif d'alimentation en eau (8, 9, 10), qui amène de l'eau à une surface supérieure de la plaque poreuse (2).

7. Simulateur de transpiration (1) selon la revendication 6,
**caractérisé en ce que**
le second dispositif d'alimentation en eau (8, 9, 10) comporte un trajet d'irrigation (8), qui court dans le bloc chauffant uniforme (3) et pénètre dans la plaque poreuse (2) dans la direction verticale et commande l'alimentation en eau vers la surface supérieure de la plaque poreuse (2), en ajustant une quantité d'eau, fournie au trajet d'irrigation (8).

8. Simulateur de transpiration (1) selon la revendication 7,
**caractérisé en ce que**
le trajet d'irrigation (8) du second dispositif d'alimentation en eau (8, 9, 10) court dans les cavités (31) du bloc chauffant uniforme (3).

9. Simulateur de transpiration (1) selon l'une quelconque des revendications 1 à 8,
**caractérisé en ce**
**qu'**il comprend un capteur de flux thermique (11), prévu entre la plaque poreuse (2) et le bloc chauffant uniforme (3) et mesure une quantité de chaleur qui traverse une unité de surface dans la direction verticale dans une unité de temps.

10. Simulateur de transpiration (1) selon l'une quelconque des revendications 1 à 9,
**caractérisé en ce que**
le dispositif de chauffage (4) comprend un dispositif de mesure, qui mesure une quantité de courant qui maintient le bloc chauffant uniforme (3) à une température constante en tant que quantité de chaleur dégagée.

11. Procédé de simulation de transpiration, fournissant de l'eau à une ou plusieurs cavités supérieures ouvertes (31) d'un bloc chauffant uniforme (3) qui est fixé en contact avec un côté inférieur d'une plaque poreuse (2), prévue horizontalement et permettant à de la vapeur de passer au travers et est chauffé par un dispositif de chauffage (4) et changeant une zone d'une surface de l'eau dans les cavités (31), pour ajuster une quantité d'évaporation depuis la plaque poreuse (2).
